(19) Europäisches Patentamt
European Patent Office
Office européen des brevets

(11) EP 4 545 587 A1

(12) **EUROPEAN PATENT APPLICATION**
published in accordance with Art. 153(4) EPC

(43) Date of publication:
**30.04.2025 Bulletin 2025/18**

(21) Application number: **23827079.7**

(22) Date of filing: **14.06.2023**

(51) International Patent Classification (IPC):
***C08G 73/02*** (2006.01) ***C08G 77/452*** (2006.01)
***A61Q 1/10*** (2006.01) ***A61Q 5/06*** (2006.01)
***A61K 8/898*** (2006.01) ***A61K 8/899*** (2006.01)

(52) Cooperative Patent Classification (CPC):
**A61K 8/898; A61K 8/899; A61Q 1/10; A61Q 5/06; C08G 73/02; C08G 77/452**

(86) International application number:
**PCT/JP2023/022082**

(87) International publication number:
**WO 2023/248893 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.06.2022 JP 2022102019**

(71) Applicant: **MITSUBISHI PENCIL COMPANY, LIMITED**
**Tokyo 140-8537 (JP)**

(72) Inventor: **YAMAZAKI Yuichi**
**Fujioka-shi, Gunma 375-8501 (JP)**

(74) Representative: **Vossius & Partner Patentanwälte Rechtsanwälte mbB**
**Siebertstrasse 3**
**81675 München (DE)**

(54) **RESIN FOR HAIR COSMETICS, AND HAIR COSMETIC**

(57) To provide a resin for hair cosmetic which is free from stickiness and highly compatible in fixing properties and combing properties without impairing performances such as water resistance, colorability and washability, and to provide a hair cosmetic. The hair cosmetics resin is obtained by bonding a poly (N-acylalkylimine) segment (b) comprising a repeating unit represented by formula (1) to at least two silicon atoms of an organopolysiloxane segment (a) constituting a main chain via an alkylene group containing a hetero atom, wherein the alkylene group containing a hetero atom is either a group represented by formula (i) or (ii), the mass average molecular weight of the segment (a) is 10,000 to 100,000, the number average molecular weight of the segment (b) is less than 800, and the mass ratio of (a) to (b) [(a)/(b)] is 58/42 to 90/10.

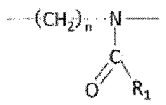
(1)

AA [In formula (1), $R_1$ represents a hydrogen atom, C1-22 alkyl group, aralkyl group, or aryl group, and n represents 2 or 3.]

(i)
$$-(CH_2)_3-NH-(CH_2)_2-NH-$$
(ii)
$$-(CH_2)_3-NH-$$

[In formula (1), $R_1$ represents a hydrogen atom, $C_{1-22}$ alkyl group, aralkyl group, or aryl group, and n represents 2 or 3.]

EP 4 545 587 A1

## Description

Technical Field

The present specification relates to a resin for hair cosmetics and a hair cosmetic that are highly compatible in both fixability and easy-combing.

Background Art

In the conventional art, in hair cosmetics used for eyelashes, head hair, and the like, various blending compositions are known, and the use form thereof is also used in various forms.

In general, a hair cosmetic of a type to be applied using an application tool such as a brush or a comb is required to have easiness of combing, no stickiness, fixability, water resistance, colorability, easiness of washing, and the like.

As known hair cosmetics, for example, there have been known:

1) a resin emulsion for an eyelash cosmetic containing a polyvinyl alcohol-based resin I and a vinyl-based resin A as a resin emulsion for an eyelash cosmetic which is excellent in water resistance, curl maintainability under high humidity, and easiness of washing with water when used in mascara and the like, wherein a weight ratio of the polyvinyl alcohol-based resin I and the vinyl-based resin A in the emulsion is 55: 45 to 99: 1, and an eyelash cosmetic containing the acryl-containing resin emulsion for an eyelash cosmetic (see, for example, Patent Document 1);
2) as an organopolysiloxane having excellent extensibility and excellent solubility/dispersibility in water or a lower alcohol, an organopolysiloxane in which a poly(N-acylalkyleneimine) segment including a repeating unit represented by a specific formula is bonded to at least two silicon atoms of an organopolysiloxane segment constituting a main chain via an alkylene group containing a hetero atom, wherein a number average molecular weight of the poly(N-acylalkyleneimine) segment is 800 to 1, 600, a mass ratio (a/b) between an organopolysiloxane segment (a) constituting the main chain and a poly(N-acylalkyleneimine) segment (b) is 65/35 to 82/18, and a weight average molecular weight of the organopolysiloxane segment constituting the main chain is 10,000 to 100,000, and a hair cosmetic containing the organopolysiloxane (see, for example, Patent Document 2); and
3) to provide a temporary hair dye excellent in colorability, water resistance, difficulty of color transfer, feeling of colored hair, absence of stickiness, and easiness of application to hair, a temporary hair dye contains, as a component (A), an organopolysiloxane in which a poly(N-acylalkyleneimine) segment including a repeating unit represented by a specific formula and having a number average molecular weight of 800 to 1,600 is bonded to at least two silicon atoms of an organopolysiloxane segment constituting a main chain having a weight average molecular weight of 10,000 to 200,000 via an alkylene group containing a hetero atom, a mass ratio of the organopolysiloxane segment to the poly(N-acylalkyleneimine) segment is 83/17 to 98/2, and a molecular weight between graft points is 12,000 to 30,000, and as the component (B), a temporary hair dye composition which contains a pigment and/or a direct dye and in which a content of the component (A) is 3 to 20 mass% (see, for example, Patent Document 3).

However, the resins for hair cosmetics of Patent Documents 1 to 3 and hair cosmetics and the like containing these resins are not sticky and are good in water resistance, colorability, easiness of washing and the like; however, when a coloring agent (coloring material) and the like contained are applied to hair, the hair is hardened by the resin for hair cosmetics (fixing resin), and there has been a case where there is a difference in texture from the surrounding hair. To solve this problem, combing is performed with a comb after application and drying; however, if the hair applied at this time is hard, the hair may be trapped when combing, a large load may be applied to the hair, and it is difficult to achieve both fixability and easy-combing.

Citation List

Patent Document

Patent Document 1: JP 2019-85366 A (Claims, Examples, etc.)
Patent Document 2: JP 2009-24114 A (Claims, Examples, etc.)
Patent Document 3: JP 2012-1480 A (Claims, Examples, etc.)

Summary of Invention

Technical Problem

The present disclosure is intended to solve the above-mentioned usual problems, and it is an object of the present disclosure to provide a hair cosmetics resin and a hair cosmetic which are free from stickiness and highly compatible in fixability and easy-combing without impairing performance such as water resistance, colorability, and easiness of washing.

Solution to Problem

As a result of intensive studies on the above-mentioned usual problems and the like, the present discloser has found that an intended hair cosmetics resin and a hair cosmetic can be obtained by setting the number average molecular weight and the like of the poly(N-acylalkylimine) segment (b) of a side chain to a predetermined value or less, in an organopolysiloxane in which a poly(N-acylalkyleneimine) segment (b) comprising a repeating unit represented by a specific formula is bonded to at least two silicon atoms of an organopolysiloxane segment (a) constituting a main chain via an alkylene group containing a hetero atom, and has completed the present disclosure.

That is, the hair cosmetics resin of the present disclosure is a resin for hair cosmetics in which the poly(N-acylalkylimine) segment (b) comprising a repeating unit represented by the following formula (1) is bonded to at least two silicon atoms of the organopolysiloxane segment (a) constituting the main chain via the alkylene group containing a hetero atom, wherein the alkylene group containing a hetero atom is any group represented by the following formulae (i) to (Vii), a mass average molecular weight of the segment (a) is 10,000 to 100,000, a number average molecular weight of the segment (b) is less than 800, and a mass ratio of (a) to (b) [(a)/(b)] is 58/42 to 90/10.

[Chem. 1]

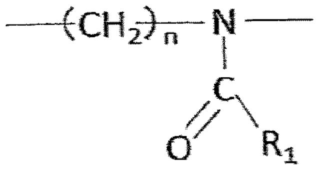

........ (1)

[In formula (1), $R_1$ represents a hydrogen atom, $C_{1-22}$ alkyl group, aralkyl group, or aryl group, and n represents 2 or 3.]

[Chem. 2]

(i)

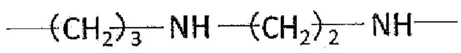

(ii)

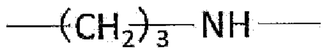

(iii)

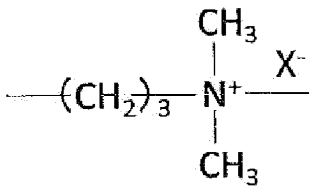

(iV)

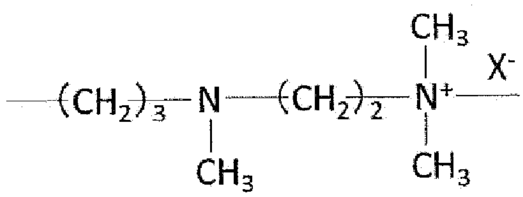

(V)

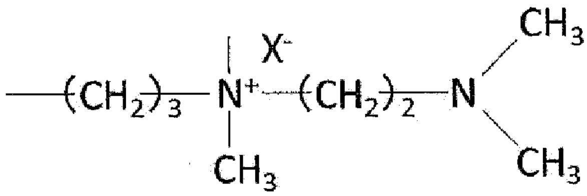

(Vi)

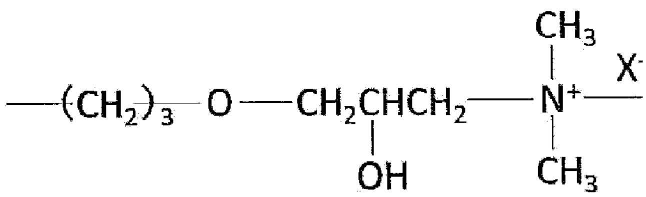

(Vii)

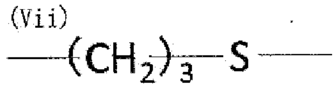

The alkylene group containing a hetero atom of the resin for hair cosmetics is preferably a group represented by the formula (i) or (ii) . The resin for hair cosmetics is preferably polyethyloxazoline amodimethicone or polysilicone-9.

The hair cosmetic of the present disclosure contains the resin for hair cosmetics having the above configuration.

Advantageous Effects of Invention

According to the present disclosure, there are provided a resin for hair cosmetics and a hair cosmetic which are free from stickiness and highly compatible in fixability and easy-combing without impairing performance such as water resistance, colorability, and easiness of washing.

Description of Embodiments

Hereinafter, embodiments of the present disclosure will be described in detail.

A hair cosmetics resin of the present disclosure is a resin for hair cosmetics in which a poly (N-acylalkylimine) segment (b) comprising a repeating unit represented by the following formula (1) is bonded to at least two silicon atoms of an organopolysiloxane segment (a) constituting a main chain via an alkylene group containing a hetero atom. In this hair cosmetics resin, the alkylene group containing a hetero atom is any group represented by the following formulae (i) to (vii), a mass average molecular weight of the segment (a) is 10,000 to 100,000, a number average molecular weight of the segment (b) is less than 800, and a mass ratio of (a) to (b), [(a)/(b)], is 58/42 to 90/10.

[Chem. 3]

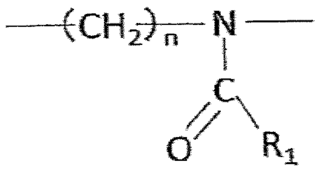

......... (1)

[In the formula (1) above, $R_1$ represents a hydrogen atom, a $C_{1}$-$_{22}$ alkyl group, an aralkyl group, or an aryl group, and n represents 2 or 3.]

[Chem. 4]

(i)

$$-(CH_2)_3-NH-(CH_2)_2-NH-$$

(ii)

$$-(CH_2)_3-NH-$$

(iii)

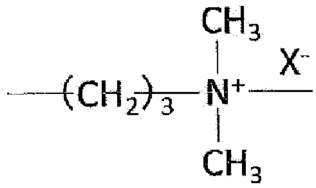

(iV)

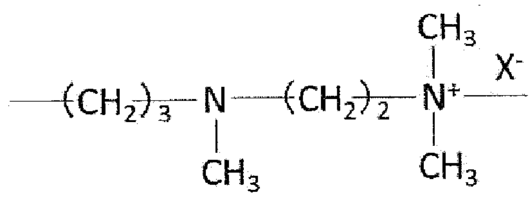

(V)

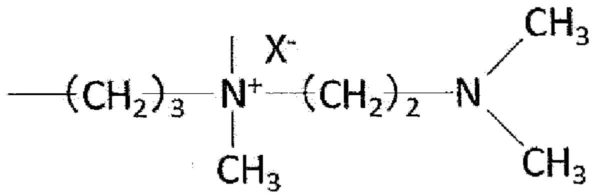

(Vi)

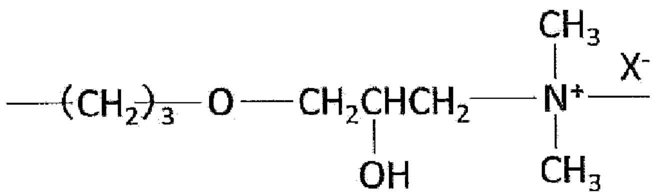

(Vii)

$$-(CH_2)_3-S-$$

In the present disclosure, at least two poly(N-acylalkyleneimine) segments may bond to any silicon atoms constituting the organopolysiloxane segment via an alkylene group containing a hetero atom; it is preferable that the poly (N-acylalkyleneimine) segments are bonded to one or more silicon atoms other than both ends via the alkylene group containing a hetero atom, and it is more preferable that the poly(N-acylalkyleneimine) segments are bonded to two or more silicon atoms other than both ends via the group.

The alkylene group containing a hetero atom functions as a linking group of the poly(N-acylalkylimine) segment (b). The alkylene group containing a hetero atom includes any group represented by the above formulae (i) to (vii), preferably a group represented by the above formula (i) or (ii), and more preferably a group represented by the above formula (i).

The N-acylalkyleneimine unit constituting the poly(N-acylalkyleneimine) segment (b) is represented by the general formula (1), examples of the alkyl group having 1 to 22 carbon atoms of $R_1$ in the general formula (1) include a linear, branched or cyclic alkyl group having 1 to 22 carbon atoms, and specific examples thereof include a methyl group, an ethyl group, a n-propyl group, an isopropyl group, a n-butyl group, a tert-butyl group, a pentyl group, a hexyl group, a

cyclohexyl group, a heptyl group, an octyl group, a nonyl group, a decyl group, an undecyl group, a dodecyl group, an octadecyl group, a nonadecyl group, an eicosyl group, and a docosyl group. Among them, an alkyl group having 1 to 10 carbon atoms, particularly 1 to 6 carbon atoms is more preferable.

Examples of the aralkyl group include an aralkyl group having 7 to 15 carbon atoms, and specific examples thereof include a benzyl group, a phenethyl group, a trityl group, a naphthylmethyl group, and an anthracenylmethyl group. Among them, an aralkyl group having 7 to 14 carbon atoms is preferable, and an aralkyl group having 7 to 10 carbon atoms is more preferable.

Examples of the aryl group include an aryl group having 6 to 14 carbon atoms, and specific examples thereof include a phenyl group, a tolyl group, a xylyl group, a naphthyl group, a biphenyl group, an anthryl group, and a phenanthryl group. Among these, an aryl group having 6 to 12 carbon atoms is preferable, and an aryl group having 6 to 9 carbon atoms is more preferable.

Among them, as $R_1$, an alkyl group having 1 to 6 carbon atoms is particularly preferable.

A mass ratio (a/b) of the organopolysiloxane segment (a) to the poly(N-acylalkyleneimine) segment (b) is 58/42 to 90/10. From the viewpoint of having both the colorability and the water resistance of a colored film as basic performance of the hair cosmetic, the soft touch , the absence of stickiness, and good easy-combing, the mass ratio is preferably 61/39 to 88/12, and more preferably 64/36 to 86/14.

In the present specification, (a) / (b) described above refers to a value obtained by dissolving the organopolysiloxane of the component (A) in deuterated chloroform by 5 mass% and determining an integral ratio of the alkyl group or the phenyl group in the organopolysiloxane segment and the methylene group in the poly(N-acylalkyleneimine) segment by nuclear magnetic resonance (1H-NMR) analysis.

The mass average molecular weight of the organopolysiloxane segment between adjacent poly(N-acylalkyleneimine) segments (molecular weight between graft points; MWg) is 1,000 to 3,500. From the viewpoint of having both the colorability and the water resistance of a colored film as basic performance of the hair cosmetic, the soft touch , the absence of stickiness, and good easy-combing, the mass average molecular weight is preferably 1,100 to 3,200, and more preferably 1,200 to 3,000.

In the present specification, the "organopolysiloxane segment between adjacent poly(N-acylalkyleneimine) segments" refers to a portion surrounded by a dashed line between two points from a bonding point (bonding point A) of the poly(N-acylalkyleneimine) segment to the organopolysiloxane segment to a bonding point (bonding point B) of the poly(N-acylalkyleneimine) segment adjacent thereto, as shown in the following formula (2), which is a segment consisting of one $R^2SiO$ unit, one $R^6$, and y+1 $R^2{}_3SiO$ units. The "poly(N-acylalkyleneimine) segment" refers to Z bonded to the $R^6$.

[Chem. 5]

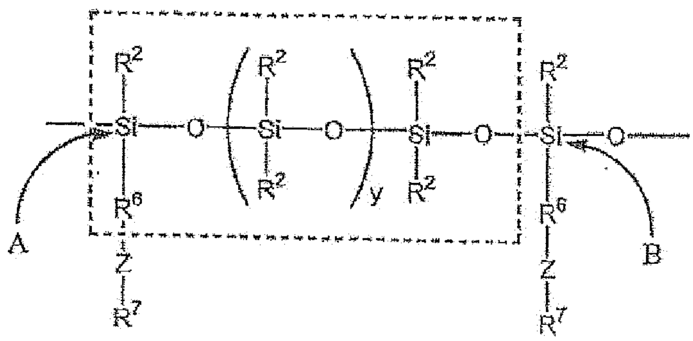

········ (2)

In the general formula (2), $R^2$ each independently represents an alkyl group having 1 to 22 carbon atoms or a phenyl group, $R^6$ represents an alkylene group containing a hetero atom, Z represents a poly(N-acylalkyleneimine) segment, $R^7$ represents a residue of a polymerization initiator, and y represents a positive number.

MWg is a molecular weight of a portion surrounded by a broken line in the general formula (2), and can be understood as a mass (g/mol) of the organopolysiloxane segment per mole of the poly(N-acylalkyleneimine) segment. When a functional group of a modified organopolysiloxane as a raw material compound is substituted by 100% with poly (N-acylalkyleneimine), MWg corresponds to a functional group equivalent (g/mol) of the modified organopolysiloxane.

The molecular weight (MWox) of the poly(N-acylalkyleneimine) segment can be measured by a method of calculating from the molecular weight of the N-acylalkyleneimine unit and the polymerization degree or by a gel permeation chromatography (GPC) measurement method described later. In the present disclosure, the molecular weight refers to a number average molecular weight measured by the GPC measurement method, and is preferably less than 800, preferably 100 or more and less than 800, and more preferably 200 or more and less than 800.

By setting the number average molecular weight to less than 800, the property of easy-combing can be improved,

and by setting the mass average molecular weight of the segment (a) to a predetermined range and setting the range of the mass ratio between (a) and (b) described above to a predetermined range, a hair cosmetics resin that highly achieves both the fixability and the easy-combing of the present disclosure is obtained. When the number average molecular weight is more than 800, the effect of the present disclosure cannot be exhibited (this point will be described in detail in Production Examples described later) .

The mass average molecular weight (MWg) of the organopolysiloxane segment can be determined by the following formula using a content (Csi) of the organopolysiloxane segment constituting the main chain.

$$MWg = (Csi \times MWox)/(100 - Csi)$$

The mass average molecular weight (MWsi) of the organopolysiloxane segment (a) constituting the main chain is 10,000 to 100,000. From the viewpoint of having both the solubility in a lower alcohol such as ethanol and the easiness of handling after dissolution, both the colorability and the water resistance of a colored film as basic performance of the hair cosmetic, the soft touch , the absence of stickiness, and good easy-combing, the mass average molecular weight is more preferably 12,000 to 80,000, and particularly preferably 14,000 to 60,000.

Since MWsi has a common skeleton with the modified organopolysiloxane as a raw material compound, MWsi is substantially the same as the mass average molecular weight of the modified organopolysiloxane as the raw material compound. The mass average molecular weight of the modified organopolysiloxane as a raw material compound is measured by GPC under the following measurement conditions and is calculated in terms of polystyrene.

Column: Two TSKgel Supre Multipore HZ-M are used side by side in series (available from Tosoh Corporation)
Eluent: 1 mM triethylamine/THF
Flow rate: 0.35 mL/min
Column temperature: 40°C
Detector: RI
Sample: 50 μL

A mass average molecular weight (MWt) of the organopolysiloxane in the present disclosure is preferably 11,000 to 170,000, more preferably 14,000 to 140,000, and still more preferably 17,000 to 95,000. As a result, a resultant has both the colorability and the water resistance of a colored film in the hair cosmetic, the soft touch , the absence of stickiness, and good easy-combing, and further has excellent solubility in a lower alcohol such as ethanol. In the present specification, MWt can be determined from the mass average molecular weight of the modified organopolysiloxane as a raw material compound and the above-described mass ratio (a/b).

The organopolysiloxane can be produced, for example, by reacting a modified organopolysiloxane represented by the following general formula (3) with a terminal-reactive poly(N-acylalkyleneimine) obtained by ring-opening polymerization of a cyclic iminoether represented by the following general formula (4).

[Chem. 6]

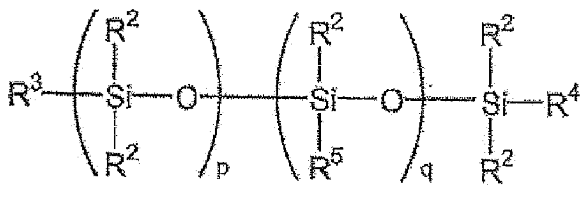

········ (3)

[In the formula (3), $R^2$ has the same meaning as described above, $R^3$ and $R^4$ each represent the same group as $R^2$, or a monovalent group represented by any of the following formulae (viii) to (xiii):

[Chem. 7]

(viii)

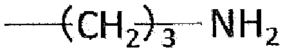

(ix)

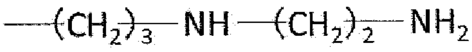

(x)

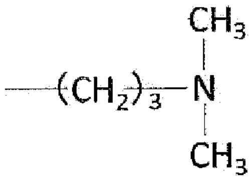

(xi)

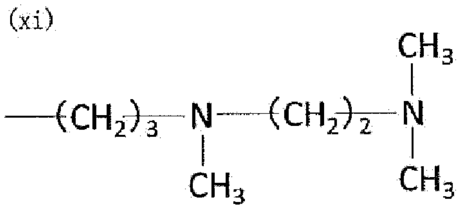

(xii)

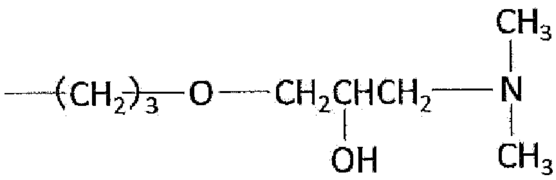

(xiii)

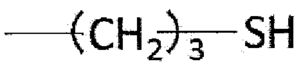

$R^5$ represents a monovalent group represented by the above formula, p represents an integer of 135 to 1,350, and q represents an integer of 3 to 57]:

[Chem. 8]

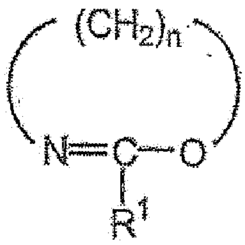

......... (4)

[In the formula (4), $R_1$ and n have the same meanings as defined above.]

The functional group equivalent of the modified organopolysiloxane is preferably 1,000 to 3,500, more preferably 1,100 to 3,200, and particularly preferably 1,200 to 3,000. The mass average molecular weight of the modified organopolysiloxane is substantially the same as the mass average molecular weight (MWsi) of the organopolysiloxane

segment (a) constituting the main chain described above.

For the ring-opening polymerization of the cyclic iminoether (4), a polymerization initiator can be used. As the polymerization initiator, a compound having strong electrophilic reactivity, for example, an alkyl ester of a strong acid such as a benzenesulfonic acid alkyl ester, a p-toluenesulfonic acid alkyl ester, a trifluoromethanesulfonic acid alkyl ester, a trifluoroacetic acid alkyl ester, or a sulfuric acid dialkyl ester can be used. Among them, dialkyl sulfate is suitably used. The amount of the polymerization initiator used is usually 1 mol of the polymerization initiator with respect to 2 to 100 mol of the cyclic iminoether (4).

As the polymerization solvent, for example, acetic acid esters such as ethyl acetate and propyl acetate, ethers such as diethyl ether, diisopropyl ether, dioxane, and tetrahydrofuran, ketones such as acetone and methyl ethyl ketone, halogen solvents such as chloroform and methylene chloride, nitrile-based solvents such as acetonitrile and benzonitrile, aprotic polar solvents such as N,N-dimethylformamide, N,N-dimethylacetamide, and dimethylsulfoxide can be used. Among them, acetic acid esters are suitably used. The amount of the solvent used is usually 20 to 2,000 parts by mass with respect to 100 parts by mass of the cyclic iminoether (4).

The polymerization temperature is usually 30 to 170°C, preferably 40 to 150°C, and the polymerization time is usually 1 to 60 hours although it is not constant depending on the polymerization temperature and the like.

For example, when 2-substituted-2-oxazoline is used as the cyclic iminoether (4), poly(N-acylethyleneimine) with n = 2 in the general formula (1) is obtained, and when 2-substituted-dihydro-2-oxazine is used, poly (N-acylpropyleneimine) with n = 3 in the general formula (1) is obtained.

Examples of the method for linking the poly(N-acylalkyleneimine) and the organopolysiloxane segment include the following methods.

1) A method of reacting a modified organopolysiloxane represented by the general formula (3) with a terminal-reactive poly(N-acylalkyleneimine) obtained by living polymerization of a cyclic iminoether
2) A reaction of forming an ester by condensation of a carboxyl group and a hydroxyl group
3) A reaction of forming an amide by condensation of a carboxyl group and an amino group
4) A reaction of forming a secondary, tertiary or quaternary ammonium between a halogenated alkyl group and a primary, secondary or tertiary amino group
5) An addition reaction of a vinyl group to an organopolysiloxane having an Si-H group
6) A reaction of forming a β-hydroxylamine between an epoxy group and an amino group

Among them, the method of the above 1) is most preferable in that the polymerization degree can be easily controlled by the use amounts of the cyclic iminoether (4) and the polymerization initiator as in the following theoretical formula [MWi is the molecular weight of poly(N-acylalkyleneimine)], and a substantially monodispersed poly(N-acylalkyleneimine) having a narrower molecular weight distribution than that of normal radical polymerization can be obtained.

MWi = [Molar number of cyclic iminoether/Molar number of polymerization initiator] × molecular weight of cyclic iminoether + molecular weight of polymerization initiator (theoretical formula )

Examples of the organopolysiloxane include poly(N-formylethyleneimine) organosiloxane, poly(N-acetylethyleneimine) organosiloxane, poly(N-propionylethyleneimine) organosiloxane, polysilicone-9, and polyethyloxazoline amodimethicone.

Particularly preferably, the alkylene group containing a hetero atom of the resin for hair cosmetics is preferably the formula (i) or (ii). More preferably, the resin for hair cosmetics is preferably polyethyloxazoline amodimethicone or polysilicone-9.

The resin for hair cosmetics of the present disclosure configured as described above is a resin for hair cosmetics obtained by bonding the poly(N-acylalkylimine) segment (b) comprising the repeating unit represented by the above formula (1) to at least two silicon atoms of the organopolysiloxane segment (a) constituting a main chain via an alkylene group containing a hetero atom, while the alkylene group containing a hetero atom is any group represented by the above formulae (i) to (vii), the mass average molecular weight of the segment (a) is 10,000 to 100,000, the number average molecular weight of the segment (b) is less than 800, and the mass ratio of (a) to (b) [(a)/(b)] is 58/42 to 90/10, whereby a water-resistant resin having suitable hardness can be produced; as a result, it is possible to obtain a hair cosmetics resin which is free from stickiness and highly compatible in both fixability and easy-combing without impairing performance such as water resistance, colorability, and easiness of washing.

[Hair cosmetic]

The hair cosmetic of the present disclosure is characterized by containing the hair cosmetics resin having the

above configuration, and preferably contains at least a pigment and a solvent in addition to the hair cosmetics resin having the above configuration.

The pigment that can be used is not particularly limited as long as it is used for hair cosmetics, and examples thereof include at least one (used singly or in mixture of two or more kinds thereof, the same applies hereinafter) of inorganic pigments, organic pigments, pearl pigments, metal powder pigments, glittering pigments, and the like.

Examples of the inorganic pigment include at least one of black pigments such as carbon black, black iron oxide, and black titanium oxide; red pigments such as iron oxide (red iron oxide), iron hydroxide, and iron titanate; brown pigments such as γ-iron oxide; yellow pigments such as yellow iron oxide and yellow earth; blue pigments such as ultramarine blue and Prussian blue; purple pigments such as manganese violet and cobalt violet; green pigments such as chromium oxide, chromium hydroxide, and cobalt titanate; or white pigments such as titanium oxide, zinc oxide, and cerium oxide. Among them, carbon black, black iron oxide, black titanium oxide, iron oxide (red iron oxide), yellow iron oxide, ultramarine blue, Prussian blue, and the like are preferable.

Examples of the organic pigment include at least one of Red No. 201, Red No. 202, Red No. 203, Red No. 204, Red No. 205, Red No. 206, Red No. 207, Red No. 208, Red No. 219, Red No. 220, Red No. 221, Red No. 228, Red No. 404, Red No. 405, Orange No. 203, Orange No. 204, Orange No. 401, Yellow No. 205, Yellow No. 401, or Blue No. 404, which are legal pigments, and among these, Red No. 202, Red No. 404, Yellow No. 205, Yellow No. 401, and Blue No. 404 are preferable.

Examples of the pearl pigment include at least one of pearl powder, bismuth oxychloride, mica, metal oxide coated mica (for example, mica titanium, iron oxide coated mica, iron oxide coated mica titanium, black iron oxide coated mica, black iron oxide coated mica titanium, yellow iron oxide coated mica, iron oxide/black iron oxide coated mica titanium, Prussian blue coated mica titanium, iron oxide/Prussian blue coated mica titanium, carmine coated mica titanium, barium sulfate coated mica titanium, and the like), metal oxide-coated alumina flakes, metal oxide-coated silica flakes, multilayer coated pearl pigment, dark blue, or the like.

Examples of the metal powder pigment include at least one of gold powder, silver powder, copper powder, aluminum powder, brass powder, or the like.

Examples of the glittering pigment include at least one of a polyethylene terephthalate aluminum epoxy laminate or a polyethylene terephthalate polyolefin laminate film.

Although a total content of these pigments varies in terms of colorability, difficulty of color transfer, feeling after application, and easiness of application, and use of the hair cosmetic (primary hair dye, mascara cosmetic, color wax, color spray), the total content is desirably 0.1 to 30 mass%, more preferably 0.5 to 20 mass%, and particularly preferably 1 to 15 mass% with respect to a total amount of the hair cosmetic.

In addition to the pigment, a surfactant, a thickener, an oil component, a polyhydric alcohol, a fragrance, a pearling agent, a dye, an ultraviolet absorber, an antioxidant, various medicinal agents (fungicides such as triclosan and trichlorocarban, anti-inflammatory agents such as dipotassium glycyrrhizinate and tocopherol acetate, and anti-dandruff agents such as zinc pyrithione and octopirox), preservatives such as methylparaben and butylparaben, pH adjusting agents such as malic acid, lactic acid, and citric acid, and the like can also be appropriately contained as necessary.

The content of the resin for hair cosmetics having the above configuration varies in terms of having both the colorability and the water resistance of a colored film as basic performance of the hair cosmetic, the soft touch , the absence of stickiness, and good easy-combing, the use of the hair cosmetic (primary hair dye, mascara cosmetic, color wax, color spray), the application form, and the like, and is desirably 5 to 30 mass%, more preferably 7 to 28 mass%, and particularly preferably 10 to 25 mass% with respect to the total amount of the hair cosmetic.

When the content of the resin for hair cosmetics is less than 5%, the fixability and the water resistance are insufficient, which is not preferable, and on the other hand, when the content is more than 30%, the resin cannot be dissolved in a solvent, the texture after hair application is deteriorated, or the easy-combing is deteriorated, which is not preferable. In the case of the aerosol type described later, the content is the "content" of a stock solution containing no propellant with respect to the total composition. The same applies hereinafter.

As the solvent that can be used, a lower alcohol, water, or a mixture of water and a lower alcohol can be used as a solvent. Examples of the lower alcohol include ethanol, 1-propanol, 2-propanol, and t-butanol, and among these, ethanol, 2-propanol, and particularly ethanol are preferable.

The content of these solvents is preferably 0.1 to 95 mass%, more preferably 10 to 90 mass%, and particularly preferably 20 to 80 mass% with respect to the total amount of the hair cosmetic from the viewpoint of pigment dispersibility, quick-drying properties after application, and the like.

The hair cosmetic of the present disclosure can contain, in addition to the above components, components usually used in hair cosmetics as long as the effects of the present disclosure are not impaired, and for example, polyhydric alcohols, surfactants, oils and/or fats, hardly volatile hydrocarbons, oily components such as silicone oils, thickeners such as hydroxyethyl cellulose, hydroxypropyl cellulose, and carboxyvinyl polymers, propellants such as LPG (liquefied petroleum gas), pentane, dimethyl ether, and diethyl ether, fragrances, preservatives, ultraviolet absorbers, chelating agents, antioxidants, plant extracts, and the like can be appropriately contained.

As the polyhydric alcohol, for example, glycols such as ethylene glycol, diethylene glycol, triethylene glycol, propylene glycol, dipropylene glycol, isoprene glycol, and 1,3-butylene glycol, for example, glycerins such as glycerin, diglycerin, and polyglycerin, and the like are preferably contained in an appropriate amount, for example, 5 mass% or less with respect to the total amount of the hair cosmetic. The content can be appropriately increased or decreased depending on the use of the hair cosmetic. For example, in the case of a dosage form such as a mascara type, a foam type, or a spray type, it is preferable to reduce the content.

The hair cosmetic of the present disclosure can be prepared by a known method, and can be produced by blending the components such as the hair cosmetics resin, a pigment, a lower alcohol, and water in the ranges of the contents and uniformly stirring and mixing the components. For example, the hair cosmetic can be prepared by stirring the hair cosmetics resin, an alcohol phase such as a lower alcohol, a pigment, water, and the like with a general purpose disperser or the like until uniform, then further adding optional components such as a pH adjuster and a thickener, stirring the components with a disperser or the like until uniform, and then stirring the components with a homomixer or the like. In addition, according to a dosage form of the hair cosmetic of the present disclosure, for example, the form of foam, hair mascara, gel, spray, cream, wax, or the like, each preferable component can be appropriately contained and prepared into each dosage form.

When the hair cosmetic of the present disclosure configured as described above is used, the hair cosmetic can be used depending on the dosage form, and when a hair application tool is used, the hair cosmetic can be used using, for example, an application tool provided with a knock-type valve device, a mascara-type application tool, a tube-type application tool, an application tool provided with a piston pressing mechanism, and the like.

Since the hair cosmetic of the present disclosure configured as described above contains the hair cosmetics resin having the above configuration, it is possible to obtain a hair cosmetic that is free from stickiness and highly compatible in both fixability and easy-combing without impairing performance such as water resistance, colorability, and easiness of washing.

## Example

Hereinafter, the present disclosure will be further described in detail with reference to production examples, examples, comparative examples, and the like. The present disclosure is not limited to the following examples and the like.

In the following production examples, the content of the organopolysiloxane segment in the resin for hair cosmetics is a value determined by a nuclear magnetic resonance method (1H-NMR), and the mass average molecular weight of a final product is a calculated value.

The molecular weight of poly(N-propionylethyleneimine) is a number average molecular weight determined by gel permeation chromatography (GPC) .

Column: Two K-804 L are used side by side in series (available from Tosoh Corporation)
Eluent: 1 mmol/dimethyldodecylamine/chloroform
Flow rate: 1. 0 mL/min
Column temperature: 40°C
Detector: RI
Sample volume: 50 μL
In terms of polystyrene

### (Production Example 1: Resin A for hair cosmetics)

8.5 g (0.055 mol) of diethyl sulfate and 42.1 g (0.43 mol) of 2-ethyl-2-oxazoline were dissolved in 101 g of dehydrated ethyl acetate, and the solution was heated at 70°C for 8 hours under a nitrogen atmosphere to synthesize a terminal-reactive poly(N-propionylethyleneimine). When the number average molecular weight was measured by GPC, the value was 770. Then 49.4 g of amodimethicone (mass average molecular weight: 20,000, amine equivalent: 1,700) was added thereto as a 33% solution of ethyl acetate at once, and the mixture was heated and refluxed for 8 hours. The reaction mixture was concentrated under reduced pressure to obtain polyethyloxazoline amodimethicone as a pale yellow rubber-like solid (92 g, yield: 92%). As a result of neutralization titration with hydrochloric acid using methanol as a solvent, it was found that no amino group remained.

### (Production Example 2: Resin B for hair cosmetics)

Poly(N-propionylethyleneimine) having a number average molecular weight of 370 was obtained from 10.6 g (0.069 mol) of diethyl sulfate, 25.0 g (0.25 mol) of 2-ethyl-2-oxazoline, and 71 g of dehydrated ethyl acetate in the same

manner as in Production Example 1 described above. In addition, 64.5 g of amodimethicone (weight average molecular weight: 20,000, amine equivalent: 1,700) was used to obtain polyethyloxazoline amodimethicone as a pale yellow rubber-like solid (94 g, yield: 94%). As a result of neutralization titration with hydrochloric acid using methanol as a solvent, it was found that no amino group remained.

(Production Example 3: Resin C for hair cosmetics)

Poly(N-propionylethyleneimine) having a number average molecular weight of 2,000 was obtained from 5.1 g (0.033 mol) of diethyl sulfate, 66.1 g (0.67 mol) of 2-ethyl-2-oxazoline, and 142 g of dehydrated ethyl acetate in the same manner as in Production Example 1 described above. In addition, 28.8 g of amodimethicone (weight average molecular weight: 20,000, amine equivalent: 1,700) was used to obtain polyethyloxazoline amodimethicone as a pale yellow rubber-like solid (95 g, yield: 95%). As a result of neutralization titration with hydrochloric acid using methanol as a solvent, it was found that no amino group remained.

(Production Example 4: Resin D for hair cosmetics)

Poly(N-propionylethyleneimine) having a number average molecular weight of 3,100 was obtained from 2.1 g (0.014 mol) of diethyl sulfate, 44.1 g (0.45 mol) of 2-ethyl-2-oxazoline, and 92 g of dehydrated ethyl acetate in the same manner as in Production Example 1 described above. In addition, 53.8 g of aminopropyl dimethicone (weight average molecular weight: 8,800, amine equivalent: 8,000) was used to obtain polysilicone-9 as a pale yellow rubber-like solid (93 g, yield: 93%). As a result of neutralization titration with hydrochloric acid using methanol as a solvent, it was found that no amino group remained.

The physical properties of the resin for hair cosmetics obtained in Production Examples 1 to 4 described above were shown in Table 1 below, and the solubility in ethanol was evaluated by the following evaluation method. The results are shown in Table 1 below.

(Solubility test in ethanol)

20 g of each of the resins for hair cosmetics obtained in Production Examples 1 to 4 described above was mixed and stirred in a container containing 100 ml of ethanol, and evaluation was performed according to the following evaluation criteria.

The results are shown in Table 1 below.

Evaluation criteria:

A: transparent
B: almost transparent and slightly cloudy
C: cloudy
D: there is a resin that is not dissolved and remains in a solid state

[Table 1]

| | Example | | Comparative Example | |
|---|---|---|---|---|
| | Resin A | Resin B | Resin C | Resin D |
| a/b | 69/31 | 82/18 | 46/54 | 74/26 |
| MWsi | 20000 | 20000 | 20000 | 8800 |
| MWox | 770 | 370 | 2000 | 3100 |
| MWg | 3400 | 3400 | 3400 | 8000 |
| MWt | 29100 | 24400 | 43500 | 7700 |
| Solubility in ethanol | B | B | B | B |

In Table 1 above, a/b, MWsi, MWox, MWg, and MWt are as follows.

a/b: mass ratio of organopolysiloxane segment (a) to poly(N-acylalkyleneimine) segment (b)
MWsi: mass average molecular weight of organopolysiloxane segment (a) constituting main chain
MWox: number average molecular weight of poly(N-acylalkyleneimine) segment (b)
MWg: mass average molecular weight of organopolysiloxane segment between adjacent poly(N-acylalkyleneimine) segments
MWt: mass average molecular weight of organopolysiloxane

[Examples 1 to 9 and Comparative Examples 1 to 3: Preparation of hair cosmetic]

Using the resins for hair cosmetics obtained in Production Examples 1 to 4 described above, hair cosmetics of Examples 1 to 9 and Comparative Examples 1 to 3 were prepared by a conventional method with a blending composition shown in Table 2 below.

The obtained hair cosmetics of Examples 1 to 9 and Comparative Examples 1 to 3 were evaluated for inhibition of coloring on hands, easy-combing, fixability, stickiness, water resistance, colorability, and washing properties by the following evaluation methods.
The results are shown in Table 2 below.

(Method of evaluating fixability)

0.2 g of each obtained hair cosmetic was applied to 1 g of human gray hair, after drying at normal temperature for 120 minutes (hereinafter, simply referred to as "after application to human gray hair and drying") , the hair was rubbed with a hand, and how much color was transferred to the hand was visually determined and evaluated according to the following evaluation criteria.

Evaluation criteria:

A: color is not transferred at all
B: color is slightly transferred but acceptable range
C: color is transferred significantly dark
D: color is transferred dark

(Method of evaluating easy-combing)

After each of the obtained hair cosmetics was applied to human gray hair and dried, a 5-grade sensory evaluation was performed by 10 panelists: a load applied to a hand when the hair was passed through a comb was evaluated by determining which was felt to be relatively larger compared with Comparative Example 1, a standard product, by 10 panelists. The property of easy-combing was evaluated with the total of the score by the 10 panelists according to the following criteria. Evaluation criteria:

5 points: smaller than Comparative Example 1
4 points: slightly smaller than Comparative Example 1
3 points: equivalent to Comparative Example 1
2 points: slightly larger than Comparative Example 1
1 point : larger than Comparative Example 1

A: 46 points or more
B: 40 points or more and less than 46 points
C: 31 points or more and less than 40 points
D: less than 31 points

(Method of evaluating stickiness)

After each of the obtained hair cosmetics was applied to human gray hair and dried, a 5-grade sensory evaluation was performed by 10 panelists: stickiness was evaluated by determining which was relatively stickier when touching a hair bundle compared with Comparative Example 1, a standard product, by 10 panelists.

Evaluation criteria:

5 points: less sticky than Comparative Example 1
4 points: slightly less sticky than Comparative Example 1
3 points: equivalent to Comparative Example 1
2 points: slightly more sticky than Comparative Example 1
1 point: more sticky than Comparative Example 1

A: 40 points or more
B: 20 points or more and less than 40 points
C: 15 points or more and less than 20 points
D: less than 15 points

(Method of evaluating water resistance)

After each of the obtained hair cosmetics was applied to human gray hair and dried, a filter paper moistened with water was pressed against the hair, and a degree of coloring of the filter paper was evaluated according to the following evaluation criteria.

Evaluation criteria:

A: not stained to filter paper at all
B: slightly stained to filter paper, but acceptable range
C: slightly darkly stained to filter paper
D: darkly stained to filter paper

(Method of evaluating colorability)

After each of the obtained hair cosmetics was applied to human gray hair and dried, the hair was visually observed, and evaluated according to the following evaluation criteria.

Evaluation criteria:

A: hair color is sufficiently changed from an original color of gray hair
B: slightly less changed than A
C: color is not significantly changed from gray hair
D: color is hardly changed from gray hair

(Method of evaluating washing properties)

After each of the obtained hair cosmetics was applied to human gray hair and dried, the hair was washed with a commercially available shampoo, and then whether the color was lost and stickiness remained was evaluated according to the following evaluation criteria.

Evaluation criteria:

A: color does not remain at all and no stickiness is observed
B: slight color or stickiness remains, but acceptable in practical use
C: color or stickiness significantly remains
D: color or stickiness remains

[Table 2]

| | Example | | | | | | | | | Comparative Example | | |
|---|---|---|---|---|---|---|---|---|---|---|---|---|
| | 1 | 2 | 3 | 4 | 5 | 6 | 7 | 8 | 9 | 1 | 2 | 3 |
| Resin A | 10 | 15 | 20 | 25 | 20 | 10 | 20 | 20 | | | | |
| Resin B | | | | | | | | | 20 | | | |
| Resin C | | | | | | | | | | | 20 | |
| Resin D | | | | | | | | | | | | 20 |
| Resin E (YUKAFORMER SM) *1 | | | | | | | | | | 20 | | |
| Pigment A (Colorona @ Dark Blue: pearl pigment) *2 | 10 | 10 | 10 | 10 | 10 | 20 | | | 10 | 10 | 10 | 10 |
| Pigment B (carbon black) | | | | | | | 1 | | | | | |
| Pigment C (Red 228) | | | | | | | | 1 | | | | |
| Water (purified water) | | | | | 5 | | | | | | | |
| Ethanol | 80 | 75 | 70 | 65 | 65 | 70 | 79 | 79 | 70 | 70 | 70 | 70 |
| Total (mass%) | 100 | 100 | 100 | 100 | 100 | 100 | 100 | 100 | 100 | 100 | 100 | 100 |
| Fixation | B | B | B | B | B | B | B | B | B | B | B | D |
| Good combability | A | A | A | A | A | A | A | A | A | D | D | D |
| Stickiness | B | B | B | B | B | B | B | B | B | A | A | D |
| Water resistance | A | A | A | A | A | B | A | A | A | A | B | B |
| Colorability | A | A | A | A | A | A | A | A | A | A | A | A |
| Washing properties | A | A | A | A | A | A | A | A | A | A | A | A |
| *1: (Methacryloyloxyethyl carboxybetaine/alkyl methacrylate) copolymer, available from OSAKA ORGANIC CHEMICAL INDUSTRY LTD.<br>*2: Available from Merck KGaA | | | | | | | | | | | | |

As shown in Table 2 above, it has been found that Example 1 to 9 within the scope of the present disclosure provide a hair cosmetics resin and a hair cosmetic which are free from stickiness and highly compatible in both fixability and easy-combing without impairing performance such as water resistance, colorability, and easiness of washing, as compared with Comparative Examples 1 to 3 outside the scope of the present disclosure.

Industrial Applicability

A hair cosmetics resin and a hair cosmetic suitable for hair cosmetics such as mascara, a hair dye, and a primary hair dye are obtained.

## Claims

1. A resin for hair cosmetics comprising a poly (N-acylalkylimine) segment (b) comprising a repeating unit represented by formula (1) bonded to at least two silicon atoms of an organopolysiloxane segment (a) constituting a main chain via an alkylene group containing a hetero atom,
   wherein the alkylene group containing a hetero atom is any group represented by formulae (i) to (vii),

a mass average molecular weight of the segment (a) is 10,000 to 100,000,
a number average molecular weight of the segment (b) is less than 800,
and a mass ratio of (a) to (b), [(a)/(b)], is 58/42 to 90/10.

[Chem. 1]

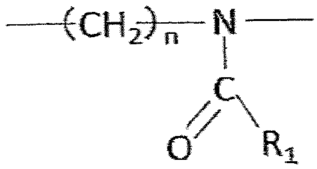

......... (1)

[In the formula (1) above, $R_1$ represents a hydrogen atom, an alkyl group having 1 to 22 carbon atoms, an aralkyl group or an aryl group, and n represents 2 or 3.]

[Chem. 2]

(i)

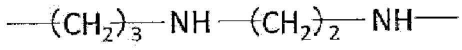

(ii)

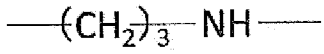

(iii)

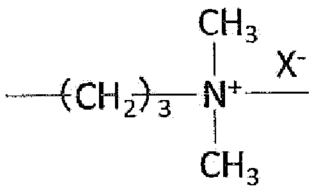

(iV)

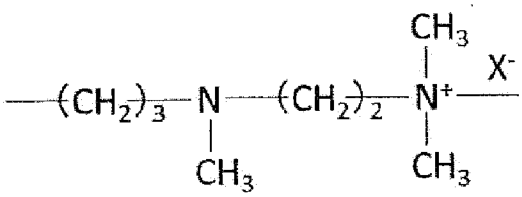

(V)

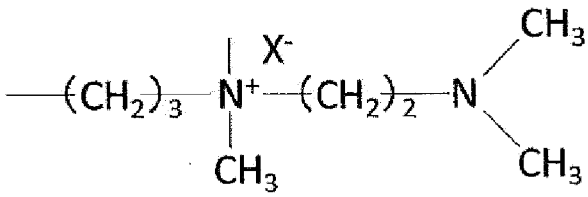

(Vi)

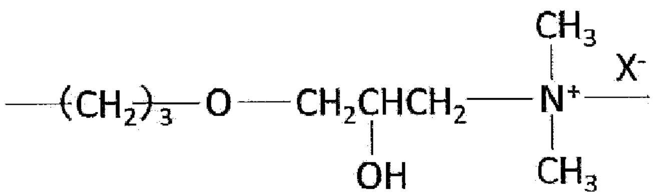

(Vii)

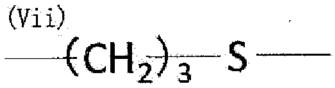

**2.** The resin for hair cosmetics according to claim 1, wherein the alkylene group containing a hetero atom of the resin for hair cosmetics is represented by a group represented by the formula (i) or (ii).

**3.** The resin for hair cosmetics according to claim 1 or 2, which is polyethyloxazoline amodimethicone or polysilicone-9.

**4.** A hair cosmetic comprising the resin for hair cosmetics according to any of claim 1 or 2.

**INTERNATIONAL SEARCH REPORT**

International application No.
**PCT/JP2023/022082**

**A. CLASSIFICATION OF SUBJECT MATTER**

***C08G 73/02***(2006.01)i; ***C08G 77/452***(2006.01)i; ***A61Q 1/10***(2006.01)i; ***A61Q 5/06***(2006.01)i; ***A61K 8/898***(2006.01)i; ***A61K 8/899***(2006.01)i

FI: A61K8/898; A61Q5/06; A61K8/899; A61Q1/10; C08G73/02; C08G77/452

According to International Patent Classification (IPC) or to both national classification and IPC

**B. FIELDS SEARCHED**

Minimum documentation searched (classification system followed by classification symbols)

C08G73/02; C08G77/452; A61Q1/10; A61Q5/06; A61K8/898; A61K8/899

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

Mintel GNPD

**C. DOCUMENTS CONSIDERED TO BE RELEVANT**

| Category* | Citation of document, with indication, where appropriate, of the relevant passages | Relevant to claim No. |
|---|---|---|
| X | JP 2016-210694 A (KAO CORP.) 15 December 2016 (2016-12-15) claims, paragraphs [0078], [0119], [0127], [0130], table 1, synthesis example 2, examples 3, 5, formulation example 1 | 1-4 |
| X | JP 4-085334 A (KAO CORP.) 18 March 1992 (1992-03-18) claims, page 7, upper left column, line 15 to upper right column, line 14, page 8, upper right column, line 17 to lower left column, line 13, page 11, lower right column, line 5 to page 13, upper left column, line 10, tables 2, 3 | 1-4 |
| A | JP 2013-023465 A (KAO CORP.) 04 February 2013 (2013-02-04) entire text | 1-4 |
| A | JP 7-133352 A (KAO CORP.) 23 May 1995 (1995-05-23) entire text | 1-4 |
| A | JP 2009-024114 A (KAO CORP.) 05 February 2009 (2009-02-05) entire text | 1-4 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 July 2023** | **15 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/022082**

| Patent document cited in search report | Publication date (day/month/year) | Patent family member(s) | Publication date (day/month/year) |
|---|---|---|---|
| JP 2016-210694 A | 15 December 2016 | (Family: none) | |
| JP 4-085334 A | 18 March 1992 | (Family: none) | |
| JP 2013-023465 A | 04 February 2013 | (Family: none) | |
| JP 7-133352 A | 23 May 1995 | EP 640643 A2<br>entire text<br>US 5747016 A | |
| JP 2009-024114 A | 05 February 2009 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019085366 A
- JP 2009024114 A
- JP 2012001480 A